# EUROPEAN PATENT APPLICATION

(11) **EP 4 245 212 A1**
(43) Date of publication of application: **20.09.2023**
(21) Application number: 22191247.0
(22) Date of filing: 19.08.2022
(51) Int. Cl.: A61B 5/00

(54) **SYSTEM AND METHOD FOR FIBER PHOTOMETRY AND OPTICAL MANIPULATION**

(30) Priority: 15.03.2022 EP 22162303
(71) Applicant: Universitätsklinikum Hamburg-Eppendorf, 20246 Hamburg (DE)
(72) Inventor: FORMOZOV, Andrey, Hamburg (DE); DIETER, Alexander, Hamburg (DE); WIEGERT, Simon, Hamburg (DE)
(74) Representative: Patentanwälte Olbricht Buchhold Keulertz

(57) **Abstract**

System for single- and multicolor fiber photometry and optical manipulation is provided, wherein the system comprises a fused fiber device 2, a light source 3 and a detector 4. The light source is a modulated or continuous light source and provides light to the fused fiber device, wherein the fused fiber device delivers this light to a brain port for excitation of a bioindicator and/or for optical manipulation (stimulation) of neuronal activity. The fiber device also receives emitted light from the excited bioindicators via the brain port and delivers it backwards to the detector. The system provides a possibility for the interrogation and readout of biological system that is expressing indicators and actuators with virtually any spectral characteristics.

## Description

The invention relates to a system and method for fiber photometry and optical manipulation. Particularly, the system and method are suitable for multicolor fiber photometry and optogenetic manipulation.

Electrically excitable cells, e.g. neurons, cardiac, striated, and smooth muscle cells, play a pivotal role in nearly all physiological functions. There have been various attempts in the past to learn about their function by the help of artificially induced excitation or inhibition and subsequent observation of their reaction, and there is a huge potential for making use of such artificial modulation for various medical purposes. Classical methods in that context are electrophysiological methods: Electrical stimulation is, for example, used for cardiac pacemaking, stimulation of particular brain areas for the relief of epileptical or parkinsonian symptoms, or targeted activation of particular signal cascades. Beside this, it is also common practice to use optical methods for monitoring and controlling activity of excitable cells. Such optical methods have gained even more importance since fluorescent biosensors have been established, such as recombinant proteins with fluorescent components. Electrophysiological and optical methods both provide particular advantages and disadvantages: Compared to electrophysiological methods, optical methods offer better spatial information and molecular specificity. On the other hand, electrophysiological methods offer high temporal resolution, allowing for direct measurements of neuronal spiking.

Fiber photometry belongs to the optical methods and is a technique of growing popularity in neuroscientific research. Typically, it is used to infer brain activity by recording calcium dynamics in genetically defined populations of neurons, and it measures bulk activity via an optical fiber in neuronal tissues. This method makes use of genetically encoded fluorescent biosensors, such as GCaMP, for example, a molecule which emits light at a certain wavelength if expressed in a cell or biological tissue and excited at a lower wavelength. Dependent on the concentration of the ligand, in this case calcium, the intensity of the emitted light is then modulated, and allows the optical measurement of intracellular calcium concentrations, and hence inference of neuronal activity. In other words, such fluorescent biosensors can function as optical indicators for neuronal activity. For that purpose, the emitted light of the biosensor is detected via a small optical fiber-based implant, connectable to an optical fiber. Today a huge palette of such fluorescent biosensors is available reaching from said GFP-based calcium-sensor GCaMP over voltage indicators for measuring membrane potential to sensors which reveal the release of various neurotransmitters, as well as sensors for neuromodulators or additional metabolic compounds such as ATP, lactate or glucose, for example.

In addition to the monitoring of biosensors, the fiber optics in the system can also be used to deliver light for activity manipulation of neurons, such as for optogenetic manipulation of neuronal activity or for infrared neural stimulation.

While fiber photometry was originally used in combination with a fluorescent Ca2+ sensitive dye in a single, column-like region of the neocortex (Adelsberger, H., Garaschuk, O., & Konnerth, A. (2005). Cortical calcium waves in resting newborn mice. Nature Neuroscience, 8(8), 988-990. https://doi.org/10.1038/nn1502), subsequent work has advanced the development of fiber photometry applications in various directions, especially after the introduction of genetically encoded calcium indicators for in vivo imaging (Cui, G., Jun, S., Jin, X. et al. Concurrent activation of striatal direct and indirect pathways during action initiation. Nature 494, 238-242 (2013). https://doi.org/10.1038/nature11846).

Later, optical fiber bundles were used to acquire photometry recordings across different brain regions simultaneously, either by distributing excitation light across different fibers with a galvanometer controlled mirror and collecting the emitted light in a single detector (Guo, Q., Zhou, J., Feng, Q., Lin, R., Gong, H., Luo, Q., Zeng, S., Luo, M., & Fu, L. (2015). Multichannel fiber photometry for population neuronal activity recording. Biomedical Optics Express, 6(10), 3919. https://doi.org/10.1364/BOE.6.003919), or by using multiple fibers simultaneously and collecting the emitted light from each optical fiber with a camera (Kim, C. K., Yang, S. J., Pichamoorthy, N., Young, N. P., Kauvar, I., Jennings, J. H., Lerner, T. N., Berndt, A., Lee, S. Y., Ramakrishnan, C., Davidson, T. J., Inoue, M., Bito, H., & Deisseroth, K. (2016). Simultaneous fast measurement of circuit dynamics at multiple sites across the mammalian brain. Nature Methods, 13(4), 325-328. https://doi.org/10.1038/nmeth.3770).

The latter approach was further extended with chronically implantable, high-density arrays of optical fibers (Sych, Y., Chernysheva, M., Sumanovski, L. T., & Helmchen, F. (2019). High-density multi-fiber photometry for studying large-scale brain circuit dynamics. Nature Methods, 16(6), 553-560. https://doi.org/10.1038/s41592-019-0400-4). Besides monitoring of different brain regions, anatomically intermingled but genetically distinct populations of neurons have also been independently measured by spectral separation of green and red calcium indicators (Meng, C., Zhou, J., Papaneri, A., Peddada, T., Xu, K., & Cui, G. (2018). Spectrally Resolved Fiber Photometry for Multi-component Analysis of Brain Circuits. Neuron, 98(4), 707-717.e4. https://doi.org/10.1016/j.neuron.2018.04.012) and voltage indicators (Marshall, J. D., Li, J. Z., Zhang, Y., Gong, Y., St-Pierre, F., Lin, M. Z., & Schnitzer, M. J. (2016). Cell-Type-Specific Optical Recording of Membrane Voltage Dynamics in Freely Moving Mice. Cell, 167(6), 1650-1662.e15. https://doi.org/10.1016/j.cell.2016.11.021).

Another example of the refinement of fiber photometry is the detection of calcium transients in subcellular compartments, such as axonal terminals in their target region (Qin, H., Lu, J., Jin, W., Chen, X., & Fu, L. (2019). Multichannel fiber photometry for mapping axonal terminal activity in a restricted brain region in freely moving mice. Neurophotonics, 6(03), 1. https://doi.org/10.1117/1.NPh.6.3.035011).

To optimally explore and integrate various combinations of the above mentioned possibilities for individual experiments, cost-efficient, open-source, and experimentally flexible systems are desired (White, S. R., Amarante, L. M., Kravitz, A. V., & Laubach, M. (2019). The Future Is Open: Open-Source Tools for Behavioral Neuroscience Research. Eneuro, 6(4), ENEURO.0223-19.2019. https://doi.org/10.1523/ENEURO.0223-19.2019). Recent developments of open-source solutions for fiber photometry have addressed individual aspects of this matter: Control systems and corresponding software to orchestrate optical illumination and signal detection of multiple spectral channels have been developed (Akam, T., & Walton, M. E. (2019). pyPhotometry: Open source Python based hardware and software for fiber photometry data acquisition. Scientific Reports, 9(1), 3521. https://doi.org/10.1038/s41598-019-39724-y; Owen, S. F., & Kreitzer, A. C. (2019). An open-source control system for in vivo fluorescence measurements from deep-brain structures.

Journal of Neuroscience Methods, 311, 170-177. https://doi.org/10.1016/j.jneumeth.2018.10.022), one of them featuring an additional, easily accessible graphical user interface (GUI) written in Python (Akam & Walton, 2019).

In a different approach, a customized system based on a lock-in amplifier has enabled fiber photometry recordings at much lower light levels as compared to commercial systems, reducing photobleaching of the biosensor (Simone, K., Füzesi, T., Rosenegger, D., Bains, J., & Murari, K. (2018). Open-source, cost-effective system for low-light in vivo fiber photometry. Neurophotonics, 5(02), 1. https://doi.org/10.1117/1.NPh.5.2.025006). Furthermore, the combination of fiber photometry recordings with EEG recordings in a customized hybrid implant was demonstrated ( Patel, A. A., McAlinden, N., Mathieson, K., & Sakata, S. (2020). Simultaneous Electrophysiology and Fiber Photometry in Freely Behaving Mice. Frontiers in Neuroscience, 14, 148. https://doi.org/10.3389/fnins.2020.00148). Finally, an open-source software platform for standardized analysis of calcium activity via fiber photometry recordings has been developed (Bruno, C. A., O'Brien, C., Bryant, S., Mejaes, J. I., Estrin, D. J., Pizzano, C., & Barker, D. J. (2021). pMAT: An open-source software suite for the analysis of fiber photometry data. Pharmacology Biochemistry and Behavior, 201, 173093. https://doi.org/10.1016/j.pbb.2020.173093).

While all of these efforts greatly reduce costs and increase accessibility of fiber photometry, they either comprise complicated assemblies of optical components or lack flexibility.

After all, a typical fiber photometry assembly is still composed of three elements: an excitation light filter, an emission light filter and a dichroic mirror for spectral separation, which need to be precisely aligned. Usually, the filters and dichroic mirrors for spectral separation are connected to several, separate optical fibers. For simultaneous measurements of multiple indicators with distinct spectra, or the combination with simultaneous optical manipulation of the neuronal tissue, the optical assembly needs to be replaced or extended with additional dichroic mirrors and filters. These systems are hard to modify to different experimental needs, and modification usually leads to increases in the cost and complexity of the system.

Nevertheless, due to the spectral characteristics of the molecular tools mentioned above, different assemblies of optical hardware are usually needed to reveal the full potential of different biosensors. In addition, the combination of multiple biosensors in one experiment often requires the investment in more complex equipment, which limits the flexibility of the experimental design. Such constraints might hamper a straightforward implementation of new molecular tools, evaluation of their performance *in vivo,* and design of new experimental paradigms; especially if the financial budget is a limiting factor.

Accordingly, it is the object of the invention to provide an enhanced system for fiber photometry, that is ready for simultaneous measurements of multiple indicators with distinct spectra and that is at the same time easy and inexpensive to handle and ease and flexible to set-up. Particularly, the system should be usable for simultaneous monitoring of multiple indicators with distinct spectral properties, and/or coupling of an additional light source for optical manipulation of tissues. Further, it is a goal of the invention to reduce the bulkiness of such a system.

The characterizing parts of claim 1 and claim 11 provide main features of the invention. Embodiments are the subject of claims 2 to 10 and 12.

As a solution to the problem mentioned above, the invention provides a system for fiber photometry and optical manipulation wherein the system comprises a fused fiber device, a light source and a detector.

The system is suitable for single color fiber photometry as well as for multicolor fiber photometry. In other words: In a first aspect, the system is a system for single color fiber photometry with the option for additional optical manipulation wherein the system comprises a fused fiber device, a light source and a detector. In a second aspect, the system is a system for multicolor fiber photometry with the option for additional optical manipulation wherein the system comprises a fused fiber device, a light source and a detector.

While the system can be used simultaneous for both purposes, i.e. for fiber photometry and optical manipulation, it can also be used for only one of these two options. In other words: In a first aspect, the system can be used for fiber photometry only. In a second aspect, the system can be used for optical manipulation only. In a third aspect, the system can be used for fiber photometry and optical manipulation simultaneously.

Optical manipulation comprises optogenetic manipulation but is not restricted to optogenetics. The fused fiber device, hereinafter abbreviated as FFC, can be a fused fiber coupler, a double-clad fiber coupler, or a fused fiber circulator. Preferably, the fused fiber device is a fused fiber coupler. Particularly preferably, the fused fiber device is a fused fiber circulator. The detector can be a photodetector, a spectrometer, a camera, or the like.

Therefore, in a preferred aspect, the system can be a system for single and/or multicolor fiber photometry and optical manipulation wherein the system comprises a fused fiber device, a light source and a detector, wherein the fused fiber devices is a fused fiber coupler, a double-clad fiber coupler, or a fused fiber circulator, and wherein the detector is a photodetector, a spectrometer or a camera. Particularly preferred is a system for multicolor fiber photometry and optogenetic manipulation wherein the system comprises a fused fiber coupler, a light source and a detector.

The system uses a single fiber optic component, namely the fused fiber device, for delivery and collection of light. In combination with fluorescent or bioluminescent indicators or even the intrinsic fluorescent properties of biological tissue and/or genetically encoded actuators, such a system allows monitoring and control of the activity of electrically excitable cells with the help of light.

Fused fiber devices are basic fiber optic devices which are used for coupling light from one or several input fibers to one or several output fibers. Such a fused fiber device - subsequently also abbreviated as FFC - is suitable to be used simultaneously for delivery of excitation and collection of emission light. For example, such a FFC has at least two ends Preferably, the FFC has three ends. By these ends the FFC is connected to a light source and a detector on the one side and to the tissue of interest on the other side.

Preferably, the FFC is made of optical fibers coated either with polyimide or metal. Such polyimide- or metal-coated fibers offer minimal autofluorescence. Reduced fiber autofluorescence will result in lower background noise and hence improve the signal to noise ratio at identical excitation power. Also it will enable recordings with higher excitation power, as the autofluorescence contribution to the over signal is decreased. Consequently, the photodetector has a higher dynamic range available for sensing indicator fluorescence. Preferably the FFC is manufactured from a so called high OH fiber type.

Further, the FFC fibers have a diameter in the range of 200 µm to 600 µm, preferably of round about 400 µm. Particularly, the FFC fibers have a diameter of round about 400 µm at the brain port. The FFC fibers can have different diameters at the other ports. In a preferred embodiment the FFC fibers do have a numerical aperture in the range of 0.22 to 0.6, preferably of 0.37-0.5.

In one example, the fused fiber device is a multimode fused fiber coupler or, synonymously, fused multimode fiber coupler. A fused multimode fiber coupler is a fused fiber coupler that has at least two communication channels. In other words, the invention provides a system for single- and multicolor fiber photometry and optogenetic manipulation, wherein the system comprises a fused multimode fiber coupler, a light source and a detector.

In a particularly preferred example, the FFC is a wideband multimode circulator. Such a circulator is characterized by higher transmittance of excitation and emission light as compared to the regular fused fiber coupler, achieved by the use of asymmetric optical fibers that are characterized by different diameters and/or NAs. Therefore, the efficiency of light collection from the indicator, which critically depends on the transmittance path to the detector, can be maximized.

It is also possible that a double-clad fiber coupler can be used as an adequate fiber device. It features from the use of asymmetrical property of fusion of double-clad fiber (single mode core surrounded by a multimode inner cladding) with a standard step-index multimode fiber. This coupler is characterized by the injection of the excitation or stimulation light from the light source into the single mode fiber component, and collection of the emission light by the double-clad core and detection at the step-index multimode fiber. In this case, the system features low autofluorescence in the excitation light path, as achieved by the use of a single mode fiber.

The light source is connected to one end of the FFC. It provides light having a wavelength that encompasses the required wavelength for excitation of the biosensor of interest and/or for optical manipulation of cellular activity in the tissue.

The detector is connected to a second end of the FFC. It receives light that is emitted by the excited biosensor and/or intrinsic tissue fluorescence. For example, the detector is a photodetector system, a spectrometer, or a camera.

In a further embodiment, the fused fiber device is a fused fiber coupler, a double-clad fiber coupler, or a fused fiber circulator, particularly a fused multimode fiber coupler, very particularly a 2x2-fiber coupler. Such a 2x2-fiber coupler has two fibers that are coupled, each of the fibers having two free ends. The 2x2-fiber coupler therefore has four ends in its default configuration. It is, however, deceivable that two or more ends are coupled to each other or that one or more of the ends are blinded, so that the 2x2-fiber coupler is based on a setting comprising four ends but has less than four functional ends. Independent of their number, each of the functional ends can either be an input end and/or an output end. Particularly, it is deceivable that one or more of the ends serves as an input end and as an output end. An input end is an end where light goes into the FFC fiber. An output end is an end where light is emitted from the FFC fiber, for example to a surrounding tissue or to a detector. Preferably at least one end of the FFC is connected to a SMA or a FC/PC connector, allowing easy coupling to a light source and/or detector. Also preferably, the length of three of the four FFC fiber ends is kept as short as possible and does not exceed 25 cm, preferably the length does not exceed 20 cm, particularly the length does not exceed 15 cm. By this, the overall fiber autofluorescence can be reduced.

In a further preferred embodiment the fused fiber device has at least one input end that is connected to the light source. The input end can also be called an input port, both terms are used synonymously hereinafter. Preferably, the input port has a SMA-connector or an FC/PC connector.

Further the fused fiber device can have at least one output end that forms a detector port. The detector port can have a SMA-connector or an FC/PC-connector.

It is particularly preferred if the fused fiber device has one end that is simultaneously working as an input end and as an output end. This end is also referred to as the brain port. The brain port can be connected to intra-cranial fiber implants at the animal's head. Preferably, the FFD end that forms the brain port has a ceramic or metal ferrule. The ferrule can be a 1.25 mm or 2.5 mm ferrule. The brain port delivers an excitation signal to the brain. The excitation signal is a light pulse that is provided by the light source to the FFD and further by the FFD to the brain port. Further, the brain port receives light emitted by excited biosensors. The FFD guides the light signal received from the brain port to the detector port. Of course, a continuous light can be used instead of the light pulse as well.

Further, the fused fiber device can have an output end connected to a dissipation port. The dissipation port serves as a dummy-end. The FFC delivers the light signal received from the light source to the brain port and to the dissipation port. The dissipation port takes up any light surplus that is not delivered from the light source via the FFD to the brain port, as it is the intrinsic property of fused fiber couplers. Thereby it prevents artifact signals that might result from backscattering or the like. As the dissipation port can be regarded as a blind end it functionally transforms the FFC into a 3-ended FFC. In other words the dissipation port can form a free end, that doesn't have any other function than the uptake of light surplus. The free end is modified to reduce backscattering of emission light. For that purpose, the free end can be covered by a cap and treated with a gel. For that purpose, the dissipation port can be equipped with a container of refractive index matching gel, or a similar material which facilitates photon emission from the optical fiber. A connector can be installed at the free end. For example the connector can be an SMA-SMA connector. Other types of connectors can be used as well. The connector is filled with refractive index matching gel. Further, the SMA-SMA connector can be closed with a black dust cap.

It is preferred, that the splitting ratio of the fused fiber device is asymmetrical. Particularly, the splitting ratio for couplers that use the same fiber diameter and numerical aperture of the fiber for the excitation (stimulation) light injection, emission light collection and detection is in the range of 70:30 to 95:05, preferably 80:20 to 95:05, particularly preferred 90:10. The splitting ratio of the wideband multimode circulator and double-clad fused fiber is asymmetrical for emission and collection as two different types of the fibers are used, providing > 85 % efficiency of excitation (stimulation) light delivery and > 75 % for emission light delivery to the detector.

Further, the light source is a switchable light source, that is switchable for multi-wavelength excitation. Particularly, the light source is switchable with very precise timing, i.e. it can be switched by less than a millisecond. Further the light source can be a TTL-controlled light source. Alternatively, the light source can be sinusoidally modulated or be a constant light source. In a preferred embodiment, the light source comprises at least one LED. Alternatively, the light source is a laser diode. It is also possible, that the light source comprises at least one multi-color LED. Preferably, the light source is directly coupled to an SMA- or FC/PC-connector of the FFD input port. In a first embodiment, the light source emits a wavelength which is between 450 and 490 nm. Alternatively, the light source emits at wavelength which is between 390 and 415 nm. Also, the light source emits at wavelength which is between 550 and 570 nm. It is particularly preferred, if the light source emits several wavelengths simultaneously or in interleaved or modulated manner, which cover all the three or even more of the aforementioned wavelength ranges. In other words, the light source emits a range of wavelength, which is at least between 390 and 415 nm, between 450 and 490 nm and/or between 550 and 570 nm.

For tailoring the system to a particular wavelength setup, it is preferred that the system comprises a filter for excitation light. This is particularly helpful, if the emission spectrum of the light source is broader than a few nanometers, as typically in the case of e.g. LEDs. This filter can also be called an excitation filter. It restricts the light that is emitted by the light source and delivered by the FFD to the brain port to a very particular range of wavelengths that are specific for the excitation of the desired biosensor or for the desired optical manipulation. For example, the first filter can be a long pass filter selected from 405/10 nm, 470/10 nm, 548/10 nm and/or 650/40 nm. Such a filter can be used for excitation of GCaMP6s, jRGECO1 a, NIR-GECO2 and/or GrabNE1h. In another example, the first filter can have a cut-off for frequencies below 575 nm, and hence can be used for optogenetic activation of ChrimsonR or other red-light sensitive opsins. In any event, the filter for excitation light can be a customized bandpass filter. A customized bandpass filter allows a greater choice of indicators also making use of different wavelength for excitation.

Further, the system comprises a filter for emission light. This filter can also shortly be called an emission filter. The emission filter is designed to block light of any wavelength that doesn't belong to the range of wavelengths emitted by the biosensor. Accordingly, it filters the light that is received from the brain port and delivered via the FFC to the detector. The emission filter can have a bandwidth selected from 530/55 nm, 535/70 nm, 575 nm and/or 700 nm. For example, it can be used for detecting the emission light of GCaMP, GrabNE1h, jRGECO1 a and/or NIR-GECO2. The emission filter can also be a double-band filter having a bandwidth of 514/28 and 603/55 nm. Alternatively, the emission filter can also be a multi-band filter having four bands, for example 440/20, 521/21, 607/34 and 700/45. The filter for emission light can be a customized bandpass filter. A customized bandpass filter allows a greater choice of indicators also making use of different wavelength for emission.

Preferably, filter holders are provided. Particularly they are provided for inserting filters in the path of the excitation light. The filter holders are small frames which can hold a disc-shaped or square-shaped filter. It is easy to exchange the filters and modify the wavelength of interest by simply inserting the suitable filter. In another embodiment the filters can be provided by the help of a filter box or can be installed permanently in the light source.

In another aspect, the invention provides a Method for fused fiber photometry comprising the steps
a. providing a system according the above described system;
b. selecting at least one fluorescent biosensor and/or at least one optogenetic actuator;
c. determine the spectral characteristics of the biosensor and the optogenetic actuator;
d. configure filter settings;
e. simultaneously delivery of excitation light and collection of emission light.

It is preferred, that the simultaneous delivery of excitation light and collection of emission light of step e comprises an asymmetrical splitting wherein 10% of the excitation light are delivered to a brain port and 90% of the excitation light are delivered to a dissipation port, if a fused fiber coupler is used. Accordingly, it is of advantage if the system that is provided in step a comprises a that is an asymmetrical FFC, providing an according splitting ratio. Further it is preferred, if the simultaneously delivery of excitation light and collection of emission light of step e comprises an asymmetrical splitting of the collection of emission light of step e comprises an asymmetrical splitting wherein at least 90% of the emission light are delivered to a detector port.

Taken together, a system for multicolor fiber photometry and optical, preferably optogenetic, manipulation is provided, wherein the system comprises a fused fiber device, a light source and a detector. Particularly, a system for multicolor fiber photometry and optical, preferably optogenetic, manipulation is provided, wherein the system comprises a fused fiber device, a light source, a detector and filters. The light source is a light source and provides light to the fused fiber device, wherein the fused fiber device delivers this light to a brain port for excitation of a bioindicator and/or for optical, preferably optogenetic, manipulation of neuronal activity. The fused fiber device also receives emitted light from the excited bioindicators via the brain port and delivers it backwards to the detector via the detector port. The system further comprises one or more filters for restricting the bandwidth of the light that is delivered from the light source via the FFC to the brain port and/or the bandwidth of the light that is emitted by the biosensor and detected by the detector.

Further features, details and advantages of the invention are apparent from the wording of the claims and from the following description of embodiments based on the drawings. Showing:
- Fig 1: Pior Art - scheme of a conventional photometry system (CPS) based on excitation and emission filters as well as dichroic mirrors;
- Fig 2: Generalized scheme of the system and method according to the invention
- Fig 3: Possible arrangements of FFP system according to the invention: with Fused Fiber Device and a detector (A),An arrangement when a spectrometer used as a detector is shown in (B).
- Fig 4.: Experimental evaluation of the FFP's arrangements with Fused Fiber Coupler and Wideband Multimode Circulator with recording of the signals in vivo (A) Experimental evaluation of the arrangement with spectrometer via measurements of the spectra of green and red fluorescent proteins (B).

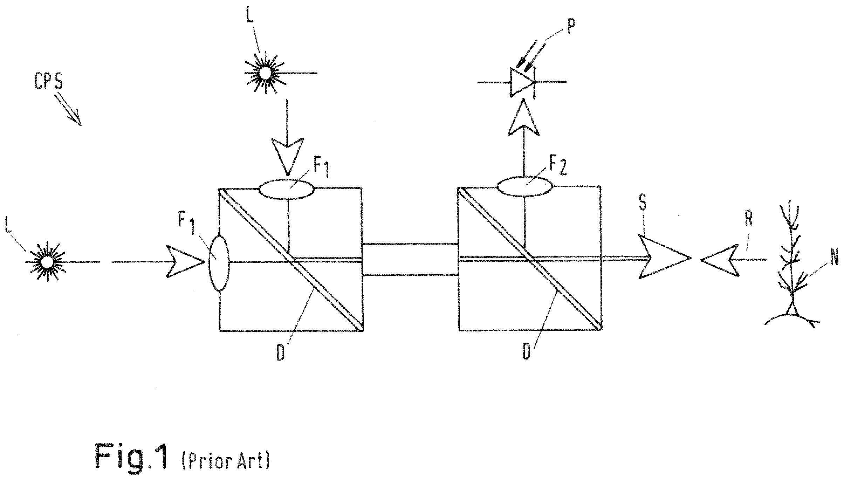

Fig. 1 shows a prior art system, namely a conventional photometry system CPS. Such a conventional system is based on several excitation and emission filters F1, F2, as well as dichroic mirrors D. A light signal S to excite the biosensor is provided by light sources L, filtered by first set of filters F1 and a set of dichroic mirrors D. In the shown example the filtered light reaches a neuronal cell N. The neuronal cell N, in turn, provides a response light signal R. The response light signal R is redirected by the dichroic mirror D via another filter F2 to the photodetector P.

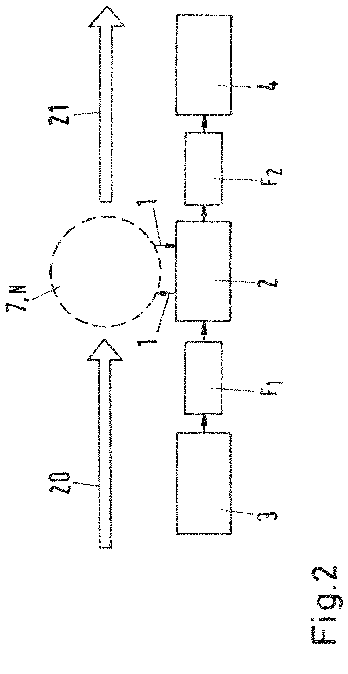

The diagram on Fig. 2 shows a schematic view of the light path in a system and method according to the invention. Light for excitation and/or stimulation is provided by a light source 3. The light source can be, for example, an LED or laser diodes with constant, modulated or interleaved excitation light, for example an LED engine, a combination of LEDs, laser diodes, one or more multicolor-LEDs or other types of light sources. The light is collimated though the excitation filter F1 and directed into the input port of the fused fiber device 2.

For the fused fiber device 2, three types of the fused fiber devices can be used: Fused Fiber Coupler, Wideband Multimode Circulator or Double-Clad Fiber Coupler.

The excitation and/or stimulation light is further directed by the brain port 1 of the fused fiber device 2 to a tissue comprising cells 9 or directly to one or more cells 9 that are expressing molecular tools for fluorescent monitoring of the activity and/or optogenetic actuators, for example to neuronal cells N. The light emitted by the fluorescent dye is redirected back to the fused fiber device 2. It passes a second set of filters F2 and is further guided to a detector 4 that can detect bulk signal in case of the solid-state detectors or PMT, or spectrally resolved information if a spectrometer is used.

The system according to the invention comprises two light paths 20, 21. A first light path 20 provides constant, modulated or interleaved excitation and/or stimulation light S to a tissue or to particular cells with genetically expressed molecular tools 9. Therein the stimulation light S is provided by the light source 3 via the filter F1 along the fused fiber device 2 to the brain port 1. The second light path 21 is the emission light path, which starts by the excited cells which provide a response signal R, namely the light emitted by the excited fluorescence molecules. Therein the response signal R reaches the fused fiber device 2 via the brain port 1 and is directed via a second set of filters F2.

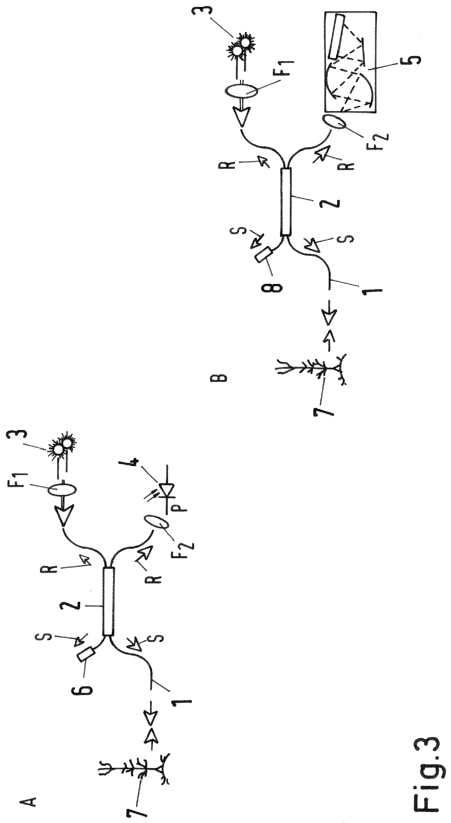

Fig 3 shows possible arrangements of an FFP system. The fused fiber device 2 can be either a fused fiber coupler, a double-clad fiber coupler or a wideband multiple circulator. As shown also in Fig. 2 the system has at least two filters F1, F2, wherein one filter F1 is a filter for the excitation light S, which is received from the light source 3. The other filter F2 is a filter for the emission light R, which is received from the stimulated cells 9. The excitation light S is guided by the fused fiber device to the brain port 1 and to a dissipation port 8. The response or emission light R is guided by the fused fiber device to the detector 4 and in part also back to the light source. The ratio, how the respective signal is distributed between brain port 1 and dissipation port 8 or detector 4 and light source 3 depends on the choosen asymmetry value of the fused fiber device. In Fig. 3B instead of the detector 4 a spectrometer 5 is used.

As shown by Fig. 3A, the excitation light is delivered into one branch of the fused fiber device 2 for FFP and split into two branches: ∼ 10 % of the power is used for the excitation of biosensors, while 90% are dissipated. As this is only an example, other split ratios are possible as well. The important aspect in splitting is that the splitting occurs asymmetrically. In the example of a splitting of 90:10, this configuration allows collection of 90% of emission light from the indicator by the photodetector through the same fiber; any backscattered excitation light is attenuated by the optical filter *F*'. The FFD is equipped with a source for excitation light and a photodetector. The excitation light is provided by a multi-color LED light source (pE-4000; CoolLED, UK), directly coupled to the FC/PC-connector of the FFD input port. Fluorescence is detected with a photodetector system (DFD_FOA_FC; Doric Lenses, Canada) coupled to the detector end via a 600 µm, 0.5 NA FC-FC fiber. In another embodiment, the 600 µm fiber becomes obsolete when replacing the SMA connector at the detector end with an FC connector, which can be directly coupled to the photodetector.

The dummy end is terminated, in order to reduce backscattering of excitation light. This is achieved by installing an SMA-SMA adapter (Thorlabs, Germany) filled with refractive index matching gel (G608N3; n ∼ 1.5 at 402 nm; Thorlabs, Germany) and closed with a black dust cap (Thorlabs, Germany). For experiments, the animal is connected to the brain end of the coupler. This connection further reduces backscattering from the brain end.

For flexible FFP recordings with different genetically encoded indicators and actuators, adequate excitation and emission filters F1, F2 are installed. The CoolLED (pE-4000) is equipped with filter-holders. The installation of emission filters is straightforward, as the photodetector can be screwed opened and is equipped with optical lenses to ensure close to perpendicular incidence of emission light on the filter surface. These lenses leave sufficient space for accommodation of an emission filter in between.

In one variant, the device, i.e. the system, used for the evaluation of FFP with FFD can be based on a commercially available 2x2 90:10 step-index multimode fiber optic coupler (for example TH400R2F2B; with SMA connectors, FP400ERT fiber type, Ø400 µm core, NA 0.5; Thorlabs, Germany), with three customized modifications: First, the coupler is originally manufactured from a FP400ERT fiber which is characterized by low content of hydroxyl groups (OH). As low OH fibers are characterized by higher absorption - and presumably autofluorescence - than high OH fibers (around wavelengths of 400 nm), the modified coupler is produced from a high OH fiber (FP400URT). Second, the length of 3 FFC branches (all except the brain end) is shortened to 15 cm, in order to reduce the total fiber length, and hence overall fiber autofluorescence. Third, the SMA connector on the brain end is exchanged for a 1.25 mm ceramic ferrule.

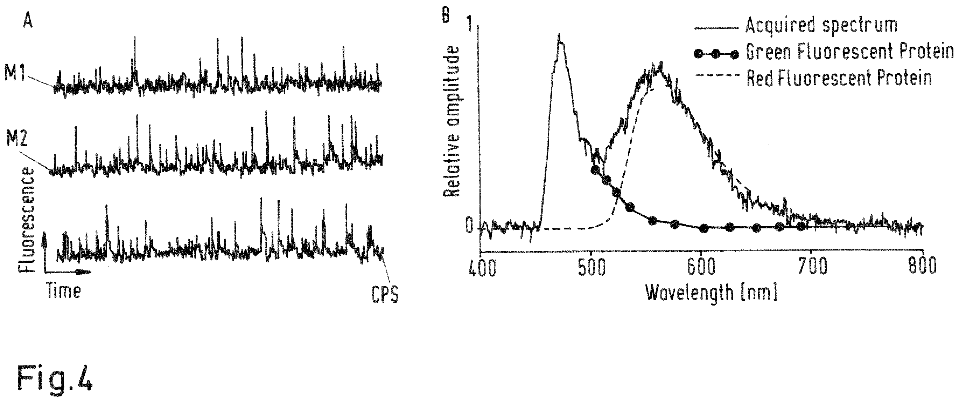

Subsequent evaluation of some of the arrangements of the invention is shown in Fig 4. Fig 4 A shows fluorescent signals measured by the detector 4 during evaluation of the system and the method with a Fused Fiber Coupler and a Wideband Multimode Circulator used as fused fiber device in comparison with a signal obtained with conventional photometry system CPS. The curve indicated by M1 shows the detected fluorescence over time while using a Fused Fiber Coupler. The curve indicated by M2 shows the detected fluorescence over time while using a Wideband Multimode Circulator. Experimental settings correspond to the arrangement presented in Fig. 3 A. For the evaluation, a population of cells in the mouse brain was modified to express the genetically encoded calcium indicator GCaMP6s in a defined brain region. The calcium transients, indicated by the peaks, of comparable quality related to brain activity can be clearly distinct in all three situations.

Experimental evaluation of the arrangement with spectrometer via simultaneous measurement of the spectra of green and red fluorescent proteins is shown in Fig. 4B. The dotted line show the curve for the green fluorescent protein. The broken line shows the curve for the red fluorescent protein. The solid line shows the acquired spectrum. Experimental settings correspond to the Fig. 3B when a spectrometer 5 is used as a detector 4. Neuronal organotypic slice culture was transfected with virus for expression of the green and red fluorophores, GCaMP6f and mRuby3. Both green and red indicators can be effectively spectrally separated from the acquired spectrum.

For the experimental examples, standard laboratory protocols, particularly handling and use of animals for the performed experiments, surgical procedures, data acquisition, data analysis and statistics where performed as described in Andrey Formozov, Alexander Dieter, J. Simon Wiegert. A flexible and versatile system for multicolor fiber photometry and optogenetic manipulation. bioRxiv 2022.03.16.484590; doi: https://doi.org/10.1101/2022.03.16.484590 , and by the not yet published application EP 22162303.6 which both are herewith incorporated in their entirety by reference.

The invention is not limited to any of the above-described embodiments, but can be modified in a variety of ways.

A system for fiber photometry recordings is proposed based on a single fused fiber device, suitable for the interrogation of neuronal circuits using fluorescent biosensors and optogenetic actuators. Application of the FFP system is not limited to neuroscientific research, but it can also be employed for other excitable cells: For example, tools for simultaneous delivery and collection of light are requested for all-optical readout and control of cardiac activity and skeletal muscles. In addition, the conceptual simplicity and potential for miniaturization may be of particular advantage when considering mobile devices, such as in research involving freely moving animals or even clinical applications. Furthermore, due to its flexibility and low cost, FFP might be useful for direct readout of optogenetically induced activity in the area of stimulation.

It is important to underline that the flexibility of the FFP system is not limited to the configurations shown herein. Instead, indicators with any combination of excitation and emission spectra can be employed, such as CFP- and YFP-based indicators, by installing adequate optical filters. This is an important advantage. Thus, the system fosters the use of the whole spectrum of available indicators which is otherwise restricted by the predetermined spectral configurations of available photometry setups.

The flexibility of the FFP system enables straightforward addition of optogenetic manipulations when choosing opsins with an activation spectrum distinct to the implemented emission filter. As shown here, the red-shifted opsin ChrimsonR can be combined with GCaMP or even NIR-GECO2. Blue-light activated opsins such as ChR2, CheRiff, or CatCh can be equally combined with red-light emitting indicators such as jRGECO1 a or jRCaMP1.

The risk for inadvertent optogenetic stimulation can be reduced with the use of higher detector gains and hence lower excitation power, ultimately being limited by photon shotnoise of the detected fluorescence. In addition, the photometry light in the system is applied in very brief pulses of 0.75 ms, at maximum rates of 130 Hz, further reducing the radiant energy of blue light arriving at the neural tissue. It is important to note that many red-shifted indicators may also be excited with blue light, which is used to excite sensors in the green range, but the obtained fluorescence is usually dimmer than the one of the green indicators. This form of cross-talk can be determined with spectroscopic measurements of the emission of each indicator in the respective bands of a multi-bandpass optical filter.

While the examples characterize the platform in the relevant spectral range of several classical indicators, any combination of excitation and emission spectra could in theory be used if the appropriate filters are installed.

However, it is important to note that the bands of the emission filters determine the fraction of the emission spectrum which propagates to the detector when combining different spectral bands in one preparation, as multi-bandpass filters typically have narrower filter bands in order to accommodate additional bands of excitation light in between the emission bands. Therefore, the gain of additional spectral bands comes at the cost of signal intensity of individual bands. Thus, in order to obtain the highest possible level of the signal, filters should be chosen for each application to maximize the overlap with the emission spectrum of each indicator.

In conclusion, the concept of the FFP system allows for simultaneous detection and manipulation of neuronal activity, with high flexibility at low cost, using widely-available and cost-effective components, while providing higher flexibility than all existing photometry systems. Flexibility can be maximized by employing a light source with a large array of spectrally distinct emitters launched into a single fiber port. Customization of the FFP system is reduced to the choice of adequate excitation and emission filters, which can be easily replaced without any previous experience in optics. This option drastically increases the degrees of freedom in the experimental design, and hence the possibilities for interrogating local neuronal populations.

All of the features and advantages, including constructional details, spatial arrangements, and process steps, arising from the claims, the description, and the drawing may be essential to the invention both individually and in a wide variety of combinations.

### List of references

- CPS: conventional photometry system
- D: dichroic mirror
- F1: Excitation filter
- F2: Emission filter
- L: light source
- N: Neuron (neuronal cell)
- P: Photodetector
- R: response light signal
- S: stimulation light signal

- 1: brain port
- 2: fused fiber device
- 3: light source
- 4: detector
- 5: spectrometer
- 6: dissipation port
- 7: cells

- 20: schematic light path, excitation and/or stimulation light path
- 21: schematic light path, emission light path

- M1: fluorescence over time detected by using a Fused Fiber Coupler
- M2: fluorescence over time detected by using a Wideband Multimode Circulator

## Claims

1. System for fiber photometry and optical manipulation wherein the system comprises a fused fiber device (2), a light source (3) and a detector (4).

2. System according to claim 1, **characterized in that** the fused fiber device is a fused fiber coupler, a double-clad fiber coupler, or a fused fiber circulator, particularly a fused multimode fiber coupler, very particularly a 2x2-fiber coupler.

3. System according to one of the aforementioned claims, **characterized in that** the fused fiber device (2) has at least one input end that is connected to the light source (3).

4. System according to one of the aforementioned claims, **characterized in that** the fused fiber device (2) has at least one output end that forms a detector port (4).

5. System according to one of the aforementioned claims, **characterized in that** the fused fiber device (2) has one end that is simultaneously working as an input end and as an output end and that forms a brain port (1).

6. System according to one of the aforementioned claims, **characterized in that the fused fiber device** (2) has an output end connected to a dissipation port.

7. System according to one of the aforementioned claims, **characterized in that** the splitting ratio of the fused fiber device (2) is asymmetrical.

8. System according to one of the aforementioned claims, **characterized in that** the light source (3) is a switchable light source, that is switchable for multi-wavelength excitation.

9. System according to one of the aforementioned claims, **characterized in that** the system comprises a filter for excitation light.

10. System according to one of the aforementioned claims, **characterized in that** the system comprises a filter for emission light.

11. Method for fused fiber photometry comprising the steps
a. providing a system according to one of the claims 1 to 10;
b. selecting at least one fluorescent biosensor and/or at least one optogenetic actuator;
c. determining the spectral characteristics of the biosensor and/or the optogenetic actuator;
d. configuring filter settings in accordance to the spectral characteristics of the chosen biosensor and/or optogenetic actuator;
e. simultaneously delivery of excitation light and collection of emission light.

12. Method according to claim 11, **characterized in that** the simultaneously delivery of excitation light and collection of emission light of step e comprises an asymmetrical splitting.
